# EUROPEAN PATENT APPLICATION

(11) **EP 4 299 723 A1**
(43) Date of publication of application: **03.01.2024**
(21) Application number: 22780612.2
(22) Date of filing: 25.03.2022
(51) Int. Cl.: C12N 5/10, A61K 35/17, A61P 35/00, C07K 14/725, C12N 15/12

(54) **GENETICALLY MODIFIED CELL AND METHOD FOR PRODUCING SAME**

(30) Priority: 31.03.2021 JP 2021059961
(71) Applicant: PHC Corporation, Toon-shi Ehime 791-0395 (JP); National Cancer Center, Tokyo 104-0045 (JP)
(72) Inventor: MATSUNO Tatsuya, Tokyo 105-8518 (JP); OKANOJO Masahiro, Tokyo 105-8518 (JP); TAKAHASHI Ryosuke, Toon-shi, Ehime 791-0395 (JP); NAKATSURA Tetsuya, Kashiwa-shi, Chiba 277-8577 (JP); SUZUKI Toshihiro, Kashiwa-shi, Chiba 277-8577 (JP)
(74) Representative: Grünecker Patent- und Rechtsanwälte PartG mbB
(86) International application number: PCT/JP2022/014558
(87) International publication number: WO 2022/210399

(57) **Abstract**

[Problem] To provide a production method for a genetically modified cell that does not require a mechanism for avoiding endogenous TCR interference. [Solution] A genetically modified cell comprising (i) an exogenous oligomeric polypeptide that is constituted from a variable region and a constant region, and (ii) an exogenous CD8 α-chain and β-chain polypeptide.

## Description

### Technical Field

The present disclosure relates to a genetically modified cell, a method for producing a genetically modified cell, and a genetically modified cell population.

### Background Art

Genetically modified T cell therapies include chimeric antigen receptor (CAR) gene-engineered T cell (CAR-T) therapy, and T cell receptor (TCR) gene-engineered T cell (TCR-T) therapy. CAR-T can target only protein antigens expressed on the surface of the cell membrane and shows complete response to B-cell blood cancers, but there are a few reports on response examples in solid cancers, which display a large degree of heterogeneity in protein antigens specifically expressed on the surface of the cancer cell membrane.

Since TCR-T targets peptides presented on HLA (human leukocyte antigen) class I on the cell surface, TCR-T can target any cancer-specific protein antigens including not only antigens on the cell membrane but also antigens in cytoplasm and nucleoproteins. TCR-T has been effective in malignant melanoma and synovial sarcoma so far.

However, in TCR-T, desired TCR genes when introduced may interfere with endogenous TCRs. The interference with endogenous TCRs not only suppresses exogenous TCR expression, but also causes an adverse event resulting from the cross-reactivity to normal cells due to the reaction with unintended peptides in some reports. There is therefore a need of techniques for avoiding mutual interference between introduced exogenous TCR genes and endogenous TCRs.

Examples of the techniques for avoiding interference with endogenous TCRs include a method for suppressing endogenous TCR expression by using RNA (ribonucleic acid) interference, and a method for suppressing expression by genetic modification using CRISPR (clustered regularly interspaced short palindromic repeat)/Cas 9 (CRISPR associated 9) or TALEN (transcription activator-like effector nuclease (Japanese Unexamined Patent Application Publication (Translation of PCT Application) No. 2020-529834, WO 2019/073964, WO 2019/073965, D. Stenger et al., Blood, September 2020, Vol. 136, Issue 12; p. 1407-1418, and L.T. Morton et al., Molecular therapy, January 2020, Vol. 28, Issue 1; p. 64-74).

### Summary of Invention

### Technical Problem

However, the method for suppressing endogenous TCR expression by using RNA interference requires some technique, such as inserting a scrambled sequence into a gene to be introduced that encodes an exogenous protein, and the finally introduced gene differs from the original base sequence. The use of CRISPR/Cas9 or TALEN may decrease the immune function because of the semi-permanent loss of endogenous TCR gene expression. To avoid interference with endogenous TCRs, there is a need of, for example, a work for editing the gene sequence of endogenous TCRs. However, techniques for producing TCR-T that do not require such a work have not been developed yet.

An object of the present invention is to provide a method for producing genetically modified cells, wherein the method does not require a mechanism for avoiding interference with endogenous TCRs.

### Solution to Problem

The inventors of the present invention have conducted intensive studies to achieve the above object and have found that genetically modified cells can be produced without using a mechanism for avoiding interference with endogenous TCRs.

Specifically, the present invention is as follows.
[1] A genetically modified cell including:
   (i) an exogenous oligomeric polypeptide having a variable region and a constant region; and
   (ii) exogenous CD8 α-chain and β-chain polypeptides.
[2] The genetically modified cell according to [1], wherein the oligomeric polypeptide is an antigen-recognizing receptor.
[3] The genetically modified cell according to [1] or [2], wherein the oligomeric polypeptide is a T cell receptor (TCR).
[4] The genetically modified cell according to any one of [1] to [3], wherein the genetically modified cell is derived from a peripheral blood mononuclear cell.
[5] A method for producing a genetically modified cell, the method including:
   introducing, into a cell of interest, the following exogenous genes:
   (i) an exogenous gene encoding an oligomeric polypeptide having a variable region and a constant region; and
   (ii) an exogenous gene encoding CD8 α-chain and β-chain polypeptides.
[6] The method according to [5], further including preparing the cell of interest from a peripheral blood mononuclear cell.
[7] The method according to [5], further including preparing the cell of interest from a peripheral blood mononuclear cell population from which CD8 positive cells have been removed.
[8] The method according to any one of [5] to [7], further including adding, to a peripheral blood mononuclear cell, at least one compound selected from the group consisting of bisphosphonate compounds and pyrophosphate monoester compounds.
[9] The method according to any one of [5] to [8], wherein the exogenous genes (i) and (ii) are introduced by electroporation.
[10] A genetically modified cell population including the genetically modified cell according to any one of [1] to [4] or the genetically modified cell produced by the method according to any one of [5] to [9].
[11] A pharmaceutical composition including the genetically modified cell population according to [10] as an active ingredient.
[12] The genetically modified cell according to any one of [1] to [4] for use in effector T cell therapy.
[13] The genetically modified cell population according to [10] for use in effector T cell therapy.

### Advantageous Effects of Invention

According to the present disclosure, genetically modified cells can be produced without using a mechanism for avoiding interference with endogenous TCRs.

### Brief Description of Drawings

FIG 1 illustrates the results of flow cytometry of γδ T cells transfected with mRNA of the TCR α-chain and β-chain in Examples;
FIG 2 illustrates a map of single-stranded mRNA including mRNA of the CD8 α-chain polypeptide and the CD8 β-chain polypeptide used in Examples;
FIG 3A illustrates the nucleic acid sequence (SEQ ID NO: 1) of the entire CD8 peptide used in Examples;
FIG 3B illustrates the nucleic acid sequence (SEQ ID NO: 2) of the CD8 α-chain polypeptide used in Examples;
FIG 3C illustrates the nucleic acid sequence (SEQ ID NO: 3) of the CD8 β-chain polypeptide used in Examples;
FIG 4 illustrates the results of flow cytometry of γδ T cells transfected with mRNA of the CD8 α-chain and β-chain in Examples;
FIG 5A illustrates the reactivity between γδ T cells transfected with the respective genes and a TCR-specific peptide, detected by Human IFN-γ ELISPOT Kit; and
FIG 5B illustrates the IFN-γ production from γδ T cells transfected with the respective genes in the presence of the TCR-specific peptide.

### Description of Embodiments

Embodiments of the present invention will be described below in detail.

Genetically modified cells according to the present invention have:
(i) an exogenous oligomeric polypeptide having a variable region and a constant region; and
(ii) exogenous CD8 α-chain and β-chain polypeptides.

Specifically, the genetically modified cells according to the present invention are produced by introducing, into cells of interest, an exogenous gene encoding an oligomeric polypeptide having a variable region and a constant region, and an exogenous gene encoding a CD8 α-polypeptide and a CD8 β-polypeptide.

As used herein, the term "oligomeric polypeptide" refers to a polypeptide composed of two or more polypeptides (i.e., two or more subunits). As used herein, the term "oligomeric polypeptide" refers to a hetero-oligomer composed of two or more different polypeptides. The number of polypeptides in the oligomeric polypeptide is not limited, and the "oligomeric polypeptide" includes a dimer composed of two polypeptides, a trimer composed of three polypeptides, a tetramer composed of four polypeptides, and oligomers composed of more polypeptides. The oligomeric polypeptide is composed of polypeptides joined together through covalent bonds, such as disulfide bonds, non-covalent bonds, such as hydrogen bonds, hydrophobic bonds, and electrostatic bonds, and intermolecular forces.

The term "exogenous" regarding a polypeptide and the gene encoding the polypeptide means that the cells of interest do not intrinsically express the polypeptide, and the cells of interest do not intrinsically have the gene. In other words, the introduction of the gene encoding an "exogenous" polypeptide means that the gene encoding the polypeptide is artificially introduced into the cells of interest. The term "endogenous" means that the cells of interest intrinsically have the gene and express the polypeptide encoded by the gene. In the case of introducing an exogenous gene, the cells of interest are preferably cells that do not intrinsically express the polypeptide encoded by the gene.

### (i) Exogenous Oligomeric Polypeptide

The exogenous oligomeric polypeptide has a variable region and a constant region. The exogenous oligomeric polypeptide is preferably an antigen-recognizing receptor.

The exogenous oligomeric polypeptide is preferably a T cell receptor (TCR). The exogenous TCR has an α-chain polypeptide and a β-chain polypeptide. The α-chain polypeptide and the β-chain polypeptide each include a variable region and a constant region. The TCR and co-receptor CD8 described below come into contact with the major histocompatibility complex (MHC) to induce the immune response. In other words, genetically modified cells having TCR as an exogenous oligomeric polypeptide can recognize target cells. In addition, the immune response induced by the TCR and CD8 binding to MHC molecules provides the cells of interest with cytotoxicity against target cells.

### (ii) Exogenous CD8 α-Chain and β-Chain Polypeptides

The genetically modified cells according to the present invention further have exogenous CD8. The CD8 has an α-chain polypeptide and a β-chain polypeptide. Having exogenous CD8 with an α-chain polypeptide and a β-chain polypeptide, the genetically modified cells have cytotoxicity.

In the related art, artificial introduction of exogenous αβ-TCR genes into αβ T cells, which are cells of interest, may have not only suppressed endogenous αβ-TCR expression because of interference with endogenous αβ-TCRs in the cells of interest, but also caused an unintended adverse event resulting from the cross-reactivity to normal cells due to mutual interference with endogenous αβ-TCRs. The inventors of the present invention have avoided the undesired event described above by using, as the cells of interest, cells that have no endogenous T cell receptor α-chain or β-chain polypeptide.

In other words, in an aspect, the genetically modified cells according to the present invention are cells that have no endogenous T cell receptor α-chain or β-chain polypeptide. The "cells that have no endogenous T cell receptor α-chain or β-chain polypeptide" may be genetically modified cells produced by using cells that are intrinsically incapable of expressing T cell receptor α-chain and β-chain polypeptides, or genetically modified cells produced by using cells that intrinsically express T cell receptor α-chain and β-chain polypeptides while suppressing the expression of these polypeptides by RNA interference or other methods. In other words, the "cells that have no endogenous T cell receptor α-chain or β-chain polypeptide" in the present invention include both genetically modified cells produced by using cells that are intrinsically incapable of expressing T cell receptor α-chain and β-chain polypeptides, and genetically modified cells in which the expression of endogenous T cell receptor α-chain and β-chain polypeptides is suppressed by using RNA interference or other methods. When the "cells that have no endogenous T cell receptor α-chain or β-chain polypeptide" are produced by using cells that are intrinsically incapable of expressing T cell receptor α-chain and β-chain polypeptides, it is not necessary to suppress the endogenous TCR expression by using a mechanism for avoiding interference with endogenous TCRs, such as RNA interference.

The genetically modified cells according to the present invention are cells that do not express endogenous T cell receptor α-chain, β-chain, or CD8 polypeptide, preferably γδ T cells. The γδ T cells are cells having endogenous T cell receptor γ-chain and δ-chain.

The mechanism of action when the genetically modified cells according to the present invention are γδ T cells will be further described. Specifically, γδ T cells normally do not express αβ-TCRs, or do not show any immune response via antigen peptides presented on MHC. However, the artificial transfection of γδ T cells with αβ-TCRs allows the γδ T cells to recognize antigen peptides via the introduced αβ-TCRs.

Since γδ T cells intrinsically do not express CD8 molecules, γδ T cells do not show cytotoxicity via antigen peptides. However, the artificial transfection of γδ T cells with CD8 as well as αβ-TCRs can provide the γδ T cells with the ability to induce cytotoxicity via antigen peptides, as in the present invention.

The artificial transfection of γδ T cells with the genes that are intrinsically expressed by αβ T cells and that induce the immune response via MHC, that is, the genes encoding αβ-TCR and CD8 molecules, can produce genetically modified cells that recognize antigen peptides via MHC and that show cytotoxicity. Note that the present invention is not intended to be bound by this theory.

The genetically modified cells according to the present invention can be produced without using a mechanism for avoiding interference with endogenous TCRs, which has been used in the related art. Specifically, the modified cells can be produced without processing or editing the endogenous gene of cells to be transfected and the exogenous nucleic acid sequences to be introduced. Having high cytotoxicity, the genetically modified cells according to the present invention are expected to be used as cell therapy for treating diseases.

A second aspect of the present invention is directed to a method for producing genetically modified cells. The production method includes introducing, into cells of interest, the following exogenous genes:
(i) an exogenous gene encoding an oligomeric polypeptide having a variable region and a constant region; and
(ii) an exogenous gene encoding CD8 α-chain and β-chain polypeptides.

In the transfection step, the genes encoding the exogenous polypeptides are introduced by any method, and a suitable method selected from known transfection methods can be used. In this step, the cells of interest are transfected with a vector carrying intended exogenous genes or transfected with nucleic acid without use of vectors. Reagents and a device commonly used for transfection can be selected according to the form of transfection.

When the cells of interest are transfected with a vector, the vector used is not limited and may be, for example, a viral vector, a plasmid vector, an artificial chromosome vector, or a transposon vector.

When the cells of interest are transfected with nucleic acid without use of vectors, the nucleic acid used is not limited and may be, for example, mRNA or DNA. Examples of transfection methods without use of vectors include an electroporation method, methods using carriers, such as liposomes and ligand-polylysine, a calcium phosphate method, and a particle gun method. In this case, for example, the exogenous genes integrated into plasmid deoxyribonucleic acid (DNA) or linear DNA or RNA are introduced into the cells of interest.

Among the above transfection methods without use of vectors, the electroporation method is preferred. In other words, the exogenous genes (i) and (ii) are preferably introduced into the cells of interest by electroporation in the production method according to the present invention. The introduction of the exogenous genes by electroporation has advantages of, for example, low chemical toxicity, low possibility of virus contamination, and stable introduction efficiency. Electroporation is preferably performed by using, for example, a suspension of cells (2 × 10⁸ cells/mL) in an electroporation buffer (Maxcyte (registered trademark, hereinafter omitted) Hyclone Buffer) and preset protocol Expanded T cell-3.

The exogenous genes can be used, for example, in the form of a vector or plasmid having the exogenous genes inserted therein such that the exogenous genes are expressed under the control of a suitable promoter. To achieve efficient gene transcription, the vector may include other regulatory sequences (e.g., an enhancer sequence and/or a terminator sequence) that work with a promoter and a transcription start site.

The method for producing genetically modified cells may further include a preparation step for preparing cells of interest before the transfection step.

The preparation step includes purification and/or expansion culture of cells of interest. The cells of interest may be prepared by isolation from a blood sample and peripheral blood mononuclear cells (PBMCs) or may be prepared by induction of differentiation from pluripotent stem cells (PSCs). In other words, the cells of interest are derived from cells selected from the group consisting of peripheral blood mononuclear cells (PBMCs) and pluripotent stem cells (PSCs). The expansion culture is carried out, for example, for 1 to 14 days, preferably for 3 to 12 days, more preferably for 5 to 7 days.

The blood sample is collected from an animal. Examples of the animal from which the blood sample is collected include, but are not limited to, human, monkey, mouse, rat, pig, and dog. In an aspect, the blood sample is collected from healthy human subjects or human patients, preferably from human patients. More preferably, the blood sample is collected from human cancer patients.

Peripheral blood mononuclear cells (PBMCs) are mononuclear cells including monocytes and lymphocytes isolated from peripheral blood, and are a type of suspension cell. Examples of the cells included in PBMCs include T cells (γδ T cells, CD4-positive T cells or CD8-positive T cells), B cells, natural killer (NK) cells, monocytes, and dendritic cells. Examples of the animal from which PBMCs are isolated include human, monkey, mouse, rat, pig, and dog. In an aspect, PBMCs are human PBMCs. PBMCs are preferably collected from human patients, more preferably from human cancer patients.

PBMC-derived cells can be confirmed by the expression of the cell type-specific marker proteins on the cell surface. Examples of known marker proteins specific to T cells include CD4, CD8, and CD3. Examples of known marker proteins specific to B cells include CD19. Examples of known marker proteins specific to NK cells include CD3, CD56, and CD94. Examples of known marker proteins specific to monocytes and dendritic cells include CD14, CD11c, and CD11c. PBMC-derived cells can also be confirmed by the expression of marker proteins common to PBMCs. Examples of known marker proteins common to peripheral blood mononuclear cells include CD45. Specifically, PBMC-derived cells can be confirmed on the basis of the following indicators:
· PBMC-derived peripheral blood mononuclear cells can be confirmed by the cells being CD45 positive (CD45 (+)).
· PBMC-derived T cells (other than γδ T cells) can be confirmed by the cells being CD4 positive (CD4 (+)), CD8 positive (CD8 (+)), CD3 positive (CD3 (+)).
· PBMC-derived B cells can be confirmed by the cells being CD19 positive (CD19 (+)).
· PBMC-derived NK cells can be confirmed by the cells being CD3 negative (CD3 (-)), CD56 positive (CD56 (+)), CD94 positive (CD94 (+)).
· PBMC-derived monocytes and dendritic cells can be confirmed by the cells being CD14 positive (CD14 (+)).
· PBMC-derived neutrophils can be confirmed by the cells being CD66b positive (CD66b (+)).

The term "positive (+)" means that the corresponding marker protein is expressed, and the term "negative (-)" means that the corresponding marker protein is not expressed.

In an aspect, the cells of interest are cells that have no endogenous T-cell receptor α-chain or β-chain polypeptide. The cells of interest are preferably γδ T cells. In other words, the genetically modified cells according to the present invention are preferably prepared by using γδ T cells. In the case of PBMC-derived γδ T cells, the PBMC-derived γδ T cells are confirmed by the expression of CD3 and γδ T cell receptor complex on the cell surface.

Preferably, the preparation step further includes preparing the cells of interest from a peripheral blood mononuclear cell population from which CD8 positive cells have been removed. Removing CD8 positive cells can provide samples having a high proportion of γδ T cells as cells of interest. More preferably, the preparation step further includes preparing the cells of interest from a peripheral blood mononuclear cell population from which CD8 positive cells and CD4 positive cells have been removed. The purity of γδ T cells can be improved by removing CD8 positive cells and CD4 positive cells.

Pluripotent stem cells (PSCs) are undifferentiated cells that have the ability to differentiate into different types of cells and the ability of self-replication, and are a type of adherent cell. Examples of PSCs include embryonic stem cells (ES cells), induced pluripotent stem cells (iPS cells), and mesenchymal stem cells (MSCs). PSCs are preferably ES cells capable of differentiating into all three germ layers, endoderm, mesoderm, and ectoderm, and artificially induced pluripotent iPS cells among the above cells. For example, in the case of using iPS cells, the cells of interest can be prepared from PBMCs after iPS cells are induced to differentiate into PBMCs. Examples of the animal from which PSCs are collected include, but are not limited to, human, monkey, mouse, rat, pig, and dog. In an aspect, PSCs are human induced pluripotent stem cells or human embryonic stem cells. PSCs are preferably human induced pluripotent stem cells.

In the preparation step, a cell activator can be used to prepare the cells of interest. For example, in the case of using γδ T cells as the cells of interest, a bisphosphonate compound and/or a pyrophosphate monoester compound can be used as γδ T cell activators. Specifically, the cells of interest can be obtained by culturing γδ T cells in the presence of these γδ T cell activators.

The bisphosphonate compound is at least one compound selected from the group consisting of pamidronate, alendronate, zoledronate, risedronate, neridronate, ibandronate, incadronate, olpadronate, solvadronate, minodronate, EB1053, etidronate, clodronate, tiludronate, and medronate. In a preferred aspect, the bisphosphonate compound is zoledronate.

The pyrophosphate monoester compound is at least one compound selected from the group consisting of isopentenyl pyrophosphate, 2-methyl-3-butenyl-1-pyrophosphate, and 4-hydroxy-3-methyl-2-butenyl-1-pyrophosphate.

In the preparation step, the culture medium used for preparing the cells of interest is not limited and can be selected from known culture media. For example, in the case of using γδ T cells as the cells of interest, the culture medium used for preparation can be selected from media commonly used for culturing blood cells, and various types of sera may be added to the culture medium. Examples of commercial media that can used for preparing γδ T cells include AIM-V (Thermo Fisher Scientific), ImmunoCult (STEMCELL Technologies), and RPMI (Roswell park memorial institute medium) 1640.

In the case of adding a serum to the culture medium, the lower limit of the concentration of serum is 1% (v/v), and the upper limit is not limited unless the serum shows cytotoxicity. For example, the concentration of the serum added to the culture medium is in the range of 1% to 50% (v/v), 2% to 30% (v/v), 3% to 20% (v/v), or 5% to 10% (v/v). Examples of the serum added to the culture medium include fetal bovine serum (FBS), human AB serum, and calf serum.

A serum-free culture medium that contains no serum can also be used. In this case, the culture medium can contain 1 to 50% (v/v) or 5 to 30% (v/v) KnockOut Serum Replacement. Examples of the serum-free culture medium include Essential 8 (Thermo Fisher), mTeSR1 (STEMCELL Technologies), StemFit series (Takara Bio Inc.), and StemFlex (Thermo Fisher Scientific).

The production method according to the present invention may further include a step of preparing a genetically modified cell population by expansion culture of the genetically modified cells transfected with the exogenous genes. The expansion culture is carried out, for example, for 1 to 14 days, preferably for 3 to 12 days, more preferably for 5 to 7 days.

In the method for producing the genetically modified cells, the genetically modified cells can be produced without using a mechanism for avoiding interference with endogenous TCRs, that is, without processing or editing the endogenous genes in the cells to be transfected. Having high cytotoxicity, the genetically modified cells produced by this production method are expected to be used as cell therapy for treating diseases.

Another aspect of the present invention is directed to a genetically modified cell population containing the genetically modified cells. The genetically modified cell population may be obtained by expansion culture of the genetically modified cells prepared by the production method described above. In this case, the expansion culture is carried out, for example, for 1 to 14 days, preferably for 3 to 12 days, more preferably for 5 to 7 days.

Another aspect of the present invention is directed to a pharmaceutical composition including, as an active ingredient, the genetically modified cells or the genetically modified cell population described above. The pharmaceutical composition shows high cytotoxicity and can be expected to be used in TCR-T therapy. The pharmaceutical composition is preferably a pharmaceutical composition for treating or preventing diseases.

A method for treating or preventing diseases is also included in the scope of the present invention. The method includes a step of administering, to a subject, an effective amount of the genetically modified cells or the genetically modified cell population described above.

The present invention further includes use of the genetically modified cells or the genetically modified cell population for producing pharmaceuticals for treating or preventing diseases.

The "diseases" regarding the present invention are diseases sensitive to the genetically modified cells according to the present invention. Examples of the diseases include, but are not limited to, cancers, hepatitis, and infectious diseases. Examples of cancers include leukemia and solid cancers. Examples of infectious diseases include influenza, acquired immunodeficiency syndrome (AIDS), tuberculosis, methicillin-resistant staphylococcus aureus (MRSA) infection, and vancomycin-resistant enterococci (VRE) infection. In addition, the genetically modified cells of the present invention can also be used in, for example, donor lymphocyte infusion for the purpose of prevention of infectious diseases after bone marrow transplantation or radiation therapy, remission of relapsed leukemia, or prevention of relapsed solid cancers after surgical resection. The "diseases" are preferably cancers, more preferably solid cancers.

The "effective amount" is the amount of the genetically modified cell population according to the present invention sufficient to show a therapeutic or preventive effect when the genetically modified cell population is administered to a subject, compared with subjects that do not receive the genetically modified cell population. The specific effective amount depends on, for example, the route of administration, the frequency of administration, and the indication, as well as the age, weight, and symptoms of the subject.

The genetically modified cells and the genetically modified cell population according to the present invention can be used in effector T cell therapy. Therefore, the present invention includes the genetically modified cells or the genetically modified cell population for use in effector T cell therapy.

In this specification, a value range expressed by using "to" indicates a range including the values before and after "to" as the minimum value and the maximum value. With regard to value ranges described stepwise in this specification, the upper limit or the lower limit of one value range may be replaced by the upper limit or the lower limit of another value range. In this specification, the term "step" includes not only an independent step but also a step that cannot be clearly distinguished from other steps as long as the step achieves a desired effect.

The present disclosure is based upon and claims the priority of the Japanese patent application described below. The following Japanese patent application is hereby incorporated by reference in its entirety: Japanese Patent Application No. 2021-059961 entitled "Genetically Modified Cell and Method for Producing Same", filed on March 31, 2021.

All documents, patent applications, and technical standards described in the present disclosure are hereby incorporated by reference in their entireties.

### Examples

### [Example 1: Preparation Step]

### Thawing of PBMC, Resting (Day 0)

A RPMI 1640+10% FBS culture medium was prepared by adding 1.5 mL FBS to 13.5 mL of RPMI 1640 in a 50-mL centrifuge tube. Cryopreserved PBMC was thawed in a 37°C water bath. After thawing of PBMC was confirmed, a sample containing the thawed PBMC was collected by using a P1000 pipette and transferred into a 15-mL centrifuge tube containing 10 mL of the heated culture medium to provide a cell suspension. The inside of a cryogenic vial was washed once by using 1 mL of the cell suspension in the 15-mL centrifuge tube. The cell suspension in the 15-mL centrifuge tube was next centrifuged at 300 × g and room temperature for 7 minutes. After centrifugation, the supernatant was removed with an aspirator, and the cells were resuspended by using 1 mL of the culture medium in the 50-mL centrifuge tube. The cell suspension was transferred to a 6-well culture plate (SUMILON (registered trademark, hereinafter omitted) Sumitomo Bakelite Co., Ltd.), and the inside of the 15-mL centrifuge tube was washed once by using 3 mL of the culture medium in the 50-mL centrifuge tube. The total content in the 15-mL centrifuge tube was transferred to the 6-well culture plate (SUMILON), and the cell suspension was allowed to stand in a 37°C-incubator for one day.

### Cell Isolation (Day 1)

Cell isolation was carried out by using a sorting system, magnetic-activated cell sorting (MACS (registered trademark, hereinafter omitted)) (Miltenyi Biotec).

The cell suspension (15 mL) that had been allowed to stand in the 37°C-incubator for one day was transferred to a 15-mL centrifuge tube and centrifuged at 300 × g and room temperature for 7 minutes. The supernatant was removed with an aspirator, and the cells were resuspended in 1 mL of MACS buffer. The cell suspension was centrifuged at 300 × g and room temperature for 7 minutes. The supernatant was removed with an aspirator, and the cells were resuspended in 80 µL of MACS buffer. CD8 beads (20 µL) vortexed for about 1 second were added to the cell suspension, and the cell suspension was allowed to stand in a refrigerator at 4°C for 15 minutes. The cell suspension was taken out of the refrigerator, and 2 mL of MACS buffer was added to the cell suspension. The cell suspension was centrifuged at 300 × g and room temperature for 7 minutes. The supernatant was removed with an aspirator, and the cells were resuspended in 500 µL of MACS buffer. A column was placed on a MACS MultiStand. After the column was rinsed once with 500 µL of MACS buffer, the cell suspension was passed through the column. The column was then washed twice with 500 µL of MACS buffer. After it was confirmed that the MACS buffer had completely passed through the column, the tube was capped and centrifuged at 300 × g and room temperature for 7 minutes.

### Induction and Stimulation of γδ T Cells (Day 2)

A reaction solution was prepared by mixing AIM-V, 10% AB Serum, Interleukin-2 (IL-2, 1000 IU/mL), and 5 µM Zoledronate, and heated in a 37°C water bath for 10 minutes. The supernatant of the centrifuged cell suspension was removed with an aspirator, and the cells were resuspended in 1 mL of AIM-V + 10% AB Serum + 5 µM Zoledronate. The number of cells was counted, and the cells were seeded in a SUMILON 12-well plate at 2 × 10⁶ cells/well in a total volume of 2.5 mL.

### Maintenance of γδ T Cells (Days 3 to 10)

Every 2 to 3 days, an equal volume of AIM-V + IL-2 (1000 IU/mL) was added to the scale of culture. When the scale of culture exceeded the scale of the culture vessel, a SUMILON 6-well plate and a SUMILON T-175 flask were used.

### [Example 2: Transfection Step]

### Electroporation Pre-treatment of γδ T Cells

Twenty four hours before electroporation, an equal volume of AIM-V + 5% AB serum + IL-2 (1000 IU/mL) was added to the scale of culture, and the cells were allowed to stand in a 37°C-incubator.

### Electroporation of yδ T Cells

All the cells were collected in a 50-mL tube, and the tube was centrifuged at 300 × g and room temperature for 7 minutes. The supernatant was removed with an aspirator, and the cells were resuspended in 10 mL of MaxCyte HyClone buffer. The tube was centrifuged at 300 × g and room temperature for 7 minutes, and MaxCyte HyClone buffer was added to reach 2.0 × 10⁸ cells/mL to prepare a cell suspension.

As an electroporation sample, single-stranded mRNA containing a nucleic acid sequence corresponding to the α-chain and the β-chain of the TCR was added to 25 µL of the cell suspension to reach a final concentration of 150 ng/µL. The total amount of the electroporation sample was added to cuvette OC-25×3 (MaxCyte, Inc), and transfection was performed by using an electroporator MaxCyte GTx (MaxCyte, Inc) and an electroporation protocol "Expanded T-cell 3".

An equal volume of serum-free RPMI 1640 culture medium was added to the cuvette, and the electroporation sample was collected. The electroporation sample was allowed to stand in a 37°C-incubator for 20 minutes after being collected, and AIM-V + 5% AB Serum + IL-2 (1000 IU/mL) was then added as a culture medium. The electroporation sample was allowed to stand in the 37°C-incubator.

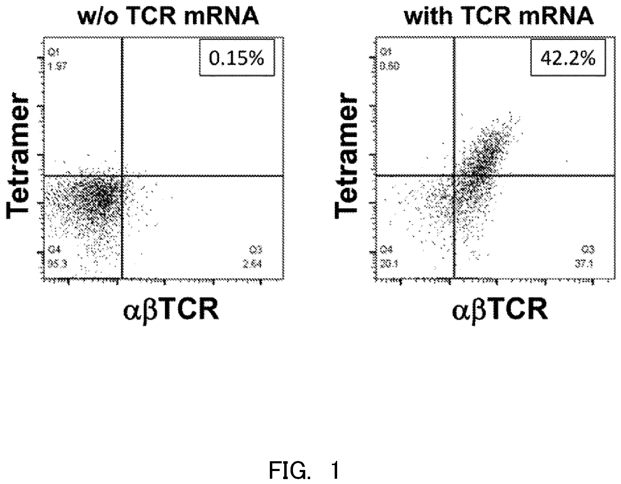

The αβ-TCR expression on the γδ T cells was analyzed by flow cytometry, and the αβ-TCR expression on the γδ T cells was observed (35.5%) as shown in FIG 1.

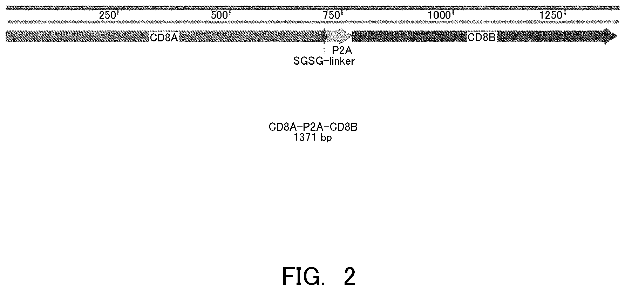

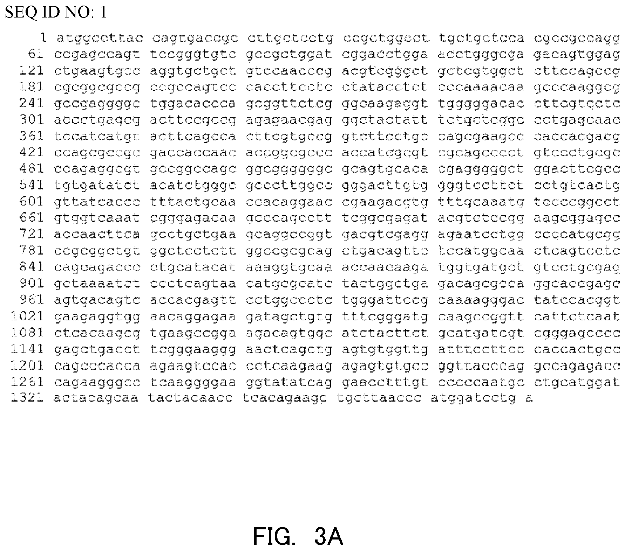

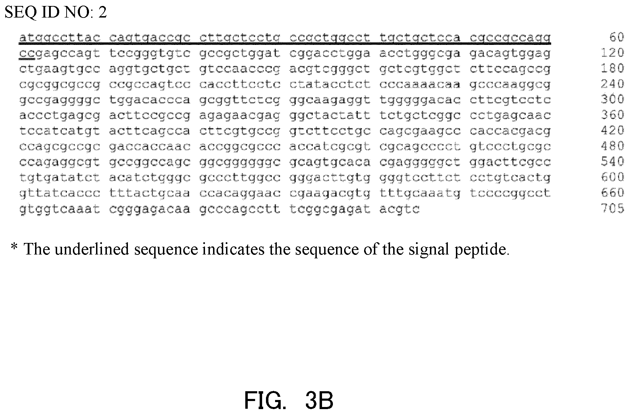

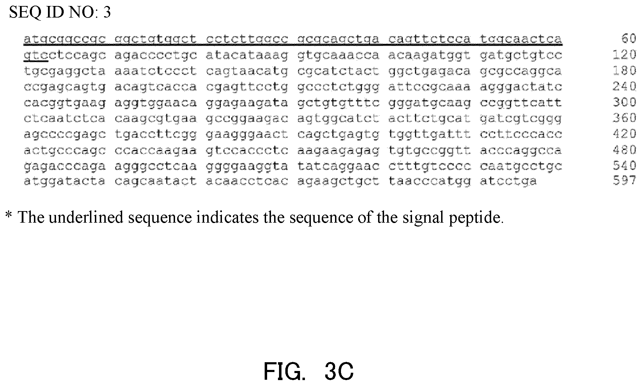

Similarly, γδ T cells were transfected with 150 ng/µL of single-stranded mRNA containing a nucleic acid sequence corresponding to the α-chain and the β-chain of the TCR and 150 ng/µL of single-stranded mRNA containing a nucleic acid sequence corresponding to the CD8 α-chain polypeptide and the CD8 β-chain polypeptide (FIG 1 and FIG 2) by electroporation to produce γδ T cells expressing αβ-TCR and CD8. The map of the single-stranded mRNA containing the nucleic acid sequence corresponding to the CD8 α-chain polypeptide and the CD8 β-chain polypeptide is illustrated in FIG 2, and the corresponding nucleic acid sequence is illustrated as SEQ ID NO: 1 in FIG 3A. The nucleic acid sequence of the CD8 α-chain polypeptide (SEQ ID NO: 2) and the nucleic acid sequence of the CD8 β-chain polypeptide (SEQ ID NO: 3) are illustrated in FIG 3B and FIG 3C, respectively.

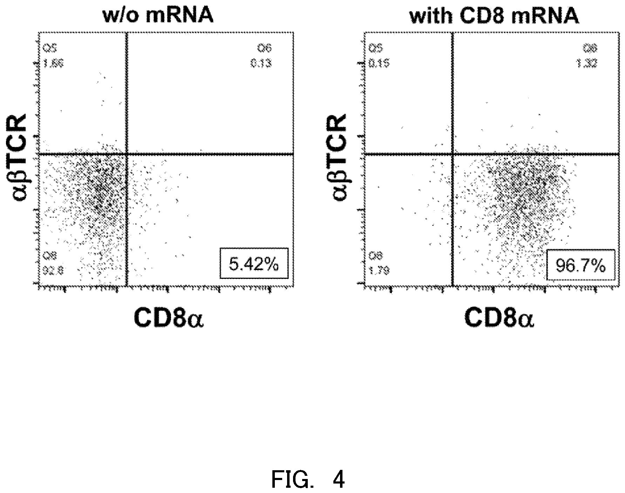

The CD8 expression on the γδ T cells was analyzed by flow cytometry, and the CD8 expression on the γδ T cells was observed (98%) as shown in FIG 4.

### [Example 3: Evaluation of Antigen-Specific Reactivity]

Next, the antigen-specific reactivity of the genetically modified γδ T cells produced based on Example 2 was evaluated by detecting interferon-γ produced upon the introduced exogenous TCR recognizing a specific peptide in Enzyme-Linked Immuno Spot (ELISPOT) assay.

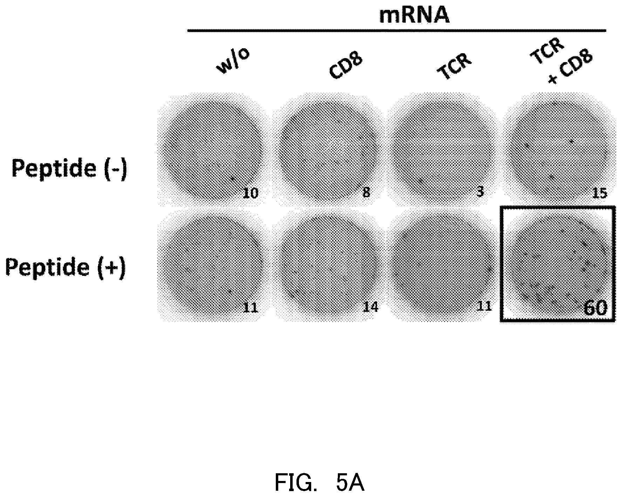

The genetically modified γδ T cells were co-cultured for 20 hours with HLA-A24 expressing T2 cells bonded to a TCR-specific peptide, which were introduced as feeder cells, and interferon-γ production was detected by using Human IFN-γ ELISPOT Kit (BD Biosciences) (FIG 5A).

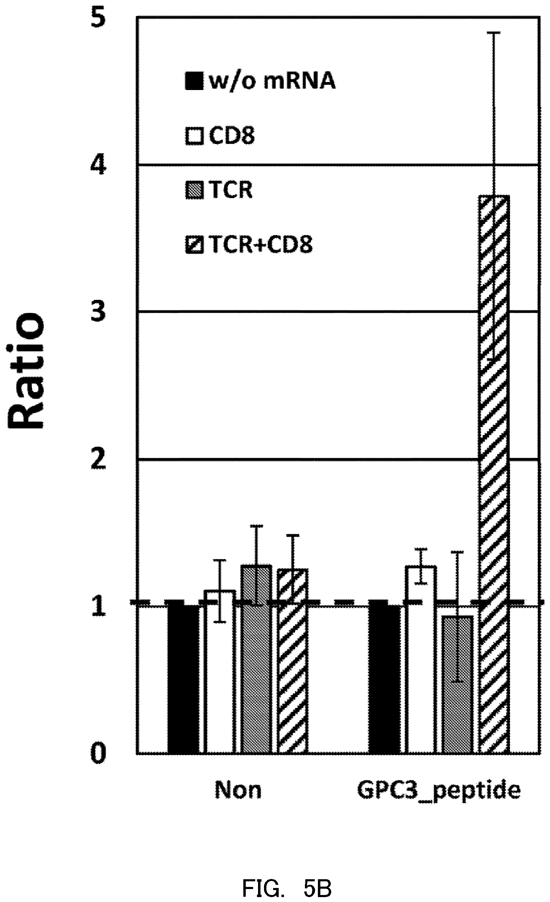

The number of spots of the negative control was subtracted from the number of spots of each sample obtained from the results of the ELISPOT assay to make correction, and the relative values when the number of spots of the non-transfected γδ T cells was set to 1 were shown (FIG 5B). In FIGS. 5A and 5B, "None", "CD8", "TCR", and "TCR+CD8" respectively represent γδ T cells not transfected with TCR or CD8, γδ T cells transfected with only CD8, γδ T cells transfected with only TCR, and γδ T cells transfected with both TCR and CD8.

The genetically modified γδ T cells transfected with CD8 showed reactivity only in the presence of the TCR-specific peptide. The genetically modified γδ T cells transfected with only one of TCR and CD8 showed no reactivity (FIGS. 5A and 5B). This indicates that the γδ T cells transfected with TCR show reactivity against the peptide when expressing CD8.

The results described above indicate that the transfection of γδ T cells with the TCR gene and the CD8 gene allows the γδ T cells to have a recognition mechanism via HLA and show antigen-specific reactivity.

## Claims

1. A genetically modified cell comprising:
(i) an exogenous oligomeric polypeptide having a variable region and a constant region; and
(ii) exogenous CD8 α-chain and β-chain polypeptides.

2. The genetically modified cell according to Claim 1, wherein the oligomeric polypeptide is an antigen-recognizing receptor.

3. The genetically modified cell according to Claim 1 or 2, wherein the oligomeric polypeptide is a T cell receptor (TCR).

4. The genetically modified cell according to any one of Claims 1 to 3, wherein the genetically modified cell is derived from a peripheral blood mononuclear cell.

5. A method for producing a genetically modified cell, the method comprising:
introducing, into a cell of interest, the following exogenous genes:
(i) an exogenous gene encoding an oligomeric polypeptide having a variable region and a constant region; and
(ii) an exogenous gene encoding CD8 α-chain and β-chain polypeptides.

6. The method according to Claim 5, further comprising preparing the cell of interest from a peripheral blood mononuclear cell.

7. The method according to Claim 5, further comprising preparing the cell of interest from a peripheral blood mononuclear cell population from which CD8 positive cells have been removed.

8. The method according to any one of Claims 5 to 7, further comprising adding, to a peripheral blood mononuclear cell, at least one compound selected from the group consisting of bisphosphonate compounds and pyrophosphate monoester compounds.

9. The method according to any one of Claims 5 to 8, wherein the exogenous genes (i) and (ii) are introduced by electroporation.

10. A genetically modified cell population comprising the genetically modified cell according to any one of Claims 1 to 4 or the genetically modified cell produced by the method according to any one of Claims 5 to 9.

11. A pharmaceutical composition comprising the genetically modified cell population according to Claim 10 as an active ingredient.

12. The genetically modified cell according to any one of Claims 1 to 4 for use in effector T cell therapy.

13. The genetically modified cell population according to Claim 10 for use in effector T cell therapy.
